# EUROPÄISCHE PATENTSCHRIFT

(11) **EP 0 703 800 B1**
(45) Veröffentlichungstag und Bekanntmachung des Hinweises auf die Patenterteilung: **25.08.1999**
(21) Anmeldenummer: 95901982.9
(22) Anmeldetag: 28.05.1994
(51) Int. Cl.: A61M 15/00, B65D 83/04

(54) **KAPSELHALTERUNG**
CAPSULE HOLDER
PORTE-CAPSULES

(30) Priorität: 03.06.1993 DE 4318455
(43) Veröffentlichungstag der Anmeldung: 03.04.1996
(60) Teilanmeldung: 98123206.9
(73) Patentinhaber: BOEHRINGER INGELHEIM INTERNATIONAL GmbH, 55218 Ingelheim am Rhein (DE); Boehringer Ingelheim Pharma KG, 55216 Ingelheim am Rhein (DE)
(72) Erfinder: HOCHRAINER, Dieter, D-55411 Bingen (DE); KINNEIR, Ross, Clifton, Bristol BS8 2UN (GB)
(74) Vertreter: Laudien, Dieter, Dr.
(86) Internationale Anmeldenummer: EP9401751
(87) Internationale Veröffentlichungsnummer: WO9428958

(56) Entgegenhaltungen:
- EP-A- 0 028 162
- FR-A- 2 216 806
- FR-A- 2 418 161
- GB-A- 2 064 334
- GB-A- 2 064 336

## Beschreibung

Die Erfindung betrifft eine Kapselhalterung für übliche Arzneimittelkapseln, insbesondere für die Verwendung in Verbindung mit Inhalationsgeräten, in denen mit mikronisiertem Arzneimittel gefüllte Kapseln benutzt werden.

Inhalationsgeräte für die Pulverinhalation, mit deren Hilfe Arzneimittelpulver aus Kapseln inhaliert werden können, sind weithin in Gebrauch. Die älteren Versionen waren so ausgelegt, daß sie nur eine Kapsel aufnehmen konnten (DE-A 14 91 715; DE-A 33 45 722).

Um dem Patienten das Nachfüllen zu erleichtern, wurden später Inhalatoren entwickelt, in denen mehrere Kapseln bereitgehalten werden können (z.B. DE-A 39 27 170). Die zur Aufnahme der Kapseln bestimmten Kammern oder Vertiefungen haben einen größeren Durchmesser als die Kapseln.

EP 0 028 162 offenbart einen Inhalator mit einem Förderband für mehrere Kapseln als integraler Bestandteil einer automatischen Öffnungsvorrichtung für die Kapseln. Das Förderband enthält Öffnungen oder Vertiefungen, die die Kapseln fest einklemmen, so daß ein zerstörungsfreies Herausnehmen der Kapseln nicht möglich ist. Zum Aufbewahren von Ersatzkapseln für ein manuelles Aufladen offenbart EP 0 028 162 auch einen becherförmigen Sammelbehälter ohne spezielle Haltevorrichtungen.

Beim Hantieren mit dem geöffneten Inhalator können die Kapseln leicht herausfallen, weil sie in den einzelnen Kammern nicht fixiert sind. Besonders beim Asthmaanfall, bei dem der Patient rasch die Inhalation vornehmen will, ist es störend, wenn die Kapseln nur lose im Inhalator liegen, so daß der Patient auch darauf achten muß, daß die Kapseln während der Benutzung nicht aus dem Gerät fallen. Andererseits ist es erwünscht, entleerte Kapseln sofort nach Gebrauch zu entfernen, ohne daß gleichzeitig die noch gefüllten Kapseln herausfallen, damit jeweils ohne weiteres festzustellen ist, wie groß der Vorrat an gefüllten Kapseln im Gerät noch ist.

Die Erfindung schlägt nun eine Kapselhalterung vor, die einerseits die Kapseln so fixiert, daß sie beim üblichen Hantieren damit nicht herausfallen, andererseits aber eine leichte Entnahme ermöglicht, und zwar unabhängig davon, mit welchem Ende die Kapseln in die Vertiefungen gesteckt werden.

Bekanntlich bestehen die zu therapeutischen Zwecken verwendeten Arzneimittelkapseln aus zwei Teilen; jeder der Teile hat zylindrische Form und hat an einem Ende einen halbkugelförmigen Abschluß. Der Innendurchmesser der beiden Zylinder ist so gewählt, daß beim Zusammenstecken mit den offenen Seiten eine relativ feste Verbindung erreicht wird. Der Außendurchmesser der beiden zylindrischen Teile ist verschieden. Bei einer handelsüblichen Kapsel der Größe 3 beispielsweise beträgt der Durchmesser des Oberteils 5,83, der Durchmesser des Unterteils 5,57 mm. Die Kapseln bestehen aus elastischem Material, vorzugsweise aus Hartgelatine.

Zylindrische Vertiefungen, die den äußeren Abmessungen der Kapseln entsprechen, lösen die erfindungsgemäße Aufgabe nicht, weil sie jeweils für eine Kapselhälfte zu eng oder zu weit sind.

Es wurde nun eine Kapselhalterung für Arzneimittelkapseln entwickelt, in welche die Kapseln sowohl mit ihrem Ober- als auch mit ihrem Unterteil gleich tief eingesteckt werden können, wobei sie einerseits ausreichend fest sitzen, um nicht herauszufallen, andererseits leicht genug entnehmbar sind, damit die Teile der Kapseln bei der Entnahme nicht versehentlich auseinandergezogen werden.

Die erfindungsgemäße Halterung besteht aus einer Vertiefung, in welche die Kapsel in Richtung ihrer Längsachse hineingesteckt wird, wobei die Wandung der Vertiefung mindestens drei parallel zur Längsachse in ungleichem Abstand voneinander angeordnete Rippen aufweist, zwischen denen die Kapsel unter Verformung eingeklemmt wird.

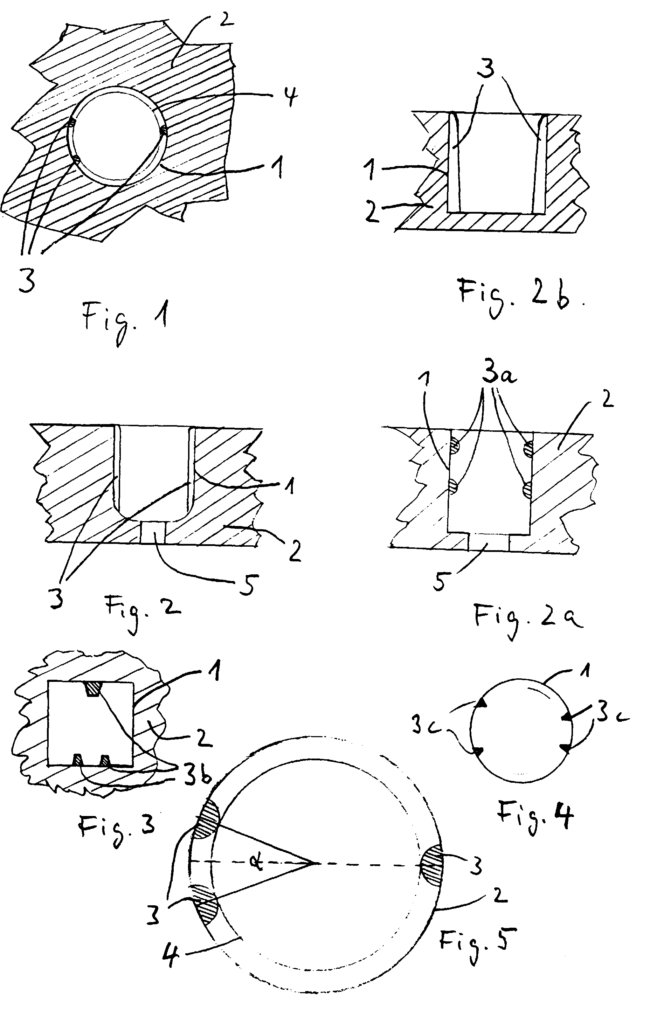

Eine Kapselhalterung dieser Art ist in den Figuren 1 bis 6 dargestellt.
Figur 1 zeigt eine erfindungsgemäße Kapselhalterung im Querschnitt.
Die Vertiefung 1 mit kreisförmigem Querschnitt in der Platte 2 weist drei Rippen 3 auf, von denen zwei verhältnismäßig nahe beeinander liegen und die dritte gegenüber. Zwischen den Rippen läßt sich ein Kreis 4 einbeschreiben, dessen Durchmesser geringfügig kleiner ist als der Außendurchmesser des unteren (dünneren) Kapselteils. Beim Einschieben zwischen die Rippen wird die Kapsel leicht verformt. Zweckmäßigerweise werden die Rippen an ihrem äußeren Ende abgerundet oder abgeschrägt, was das Einführen der Kapseln erleichtert und eine Beschädigung der Kapseln beim Einführen verhindert.

Der Querschnitt der Rippen läßt viele Abwandlungen zu, meist ist er halbkreisförmig bzw. abgerundet, er kann aber auch z.B. dreieckig oder viereckig sein, wobei die Höhe der Rippen vorzugsweise gleich ist, aber nicht gleich sein muß. Entsprechendes gilt für die Breite der Rippen. Die Fläche, mit der sich Kapseln und Rippen berühren, sollte relativ klein sein, damit auch nach längerem Aufbewahren in der Halterung die Kapsel noch gut entnommen werden kann und nicht verklebt. Die Vertiefung kann sich (bei konstanter Rippenhöhe) etwas verengen, das heißt leicht konisch geformt sein; auch können die Rippen zum unteren Ende der Vertiefung hin etwas höher werden, so daß auch in diesem Fall (bei konstantem Querschnitt der Vertiefung) eine Verringerung des Zwischenraums zwischen den Rippen erreicht wird.

Figur 2 zeigt einen senkrechten Schnitt entlang der Längsachse einer erfindungsgemäßen Kapselhalterung, wobei in der Vertiefung 1 der Platte 2 die Rippen 3 zu erkennen sind. Die Vertiefung 1 besitzt im allgemeinen unten eine Öffnung 5, welche die Reinigung erleichtert.

Wie Figur 2a zeigt, können die Rippen auch durch entsprechend angeordnete Noppen 3a ersetzt werden.

In Figur 2b wächst die Höhe des Rippenquerschnitts nach unten, so daß sich nach unten hin ein geringerer Abstand der Rippen voneinander ergibt.

Figur 3 zeigt ein Beispiel für eine Kapselhalterung mit rechteckigem Querschnitt und einer andersartigen Gestaltung der Rippen (3b).

Eine größere Zahl von Rippen läßt Figur 4 erkennen, wobei die Rippen 3c einen dreieckigen Querschnitt haben.

Um (unter sonst gleichen Voraussetzungen) eine ausreichende Verformung zu ermöglichen, sind die Rippen - bezogen auf den einbeschriebenen Kreis - nicht in gleichen Abständen angeordnet. Dies soll in Figur 5 veranschaulicht werden. Der Winkel α zwischen den beiden enger benachbarten Rippen 3 beträgt etwa 30 bis 60°, vorzugsweise 35 bis 50°. Besonders bewährt sich ein Winkel von ca. 40°. Die gegenüberliegende Rippe liegt auf dem Durchmesser, der den Winkel α halbiert oder ist allenfalls geringfügig seitlich versetzt. Bei einem Winkel von z. B. 40° zentriert sich die Kapsel beim Einführen von selbst, während sie bei zu kleinem Winkel schräg stehen kann, so daß u. U. das herausnehmen behindert sein kann, insbesondere wenn mehrere Halterungen dicht nebeneinander vorgesehen sind.

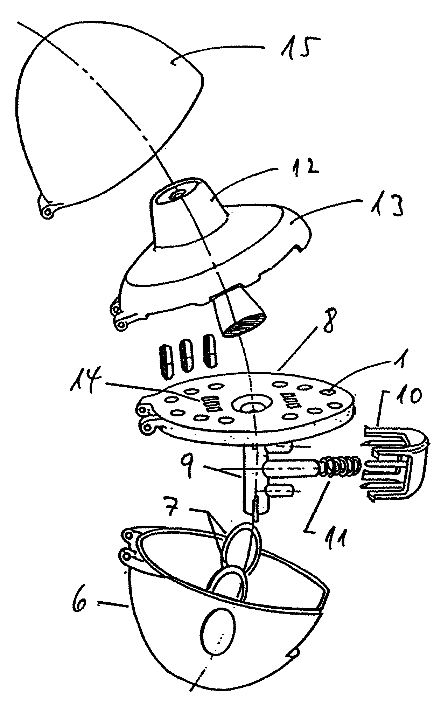

Aus Figur 6 ist zu entnehmen, wie ein Inhalator aufgebaut sein kann, in den erfindungsgemäße Kapselhalter integriert sind.

In einem Unterteil 6 mit zwei Fenstern 7 befindet sich die Platte 8, die mit der Inhalationskammer 9 verbunden ist und in der sich auch 12 Kapselhalterungen 1 befinden. Zum Öffnen der Kapseln in der Kapselkammer 9 dient der mit zwei speziell geschliffenen Nadeln versehene Knopf 10, der gegen den Druck der Feder 11 eingedrückt wird und dabei die Kapsel in der Kammer an zwei Stellen aufschneidet bzw. aufsticht. Beim Inhalieren durch das Gerät mittels des Mundrohrs 12, das mit dem Oberteil 13 verbunden ist, gelangt die Luft durch die Öffnungen 14 in das Unterteil 6 und von dort am unteren Ende in die Kapselkammer 9, die so gebaut ist wie die Kapselkammer in dem Inhalator gemäß DE-A 3927170. Das Gerät wird durch einen Deckel verschlossen, der klappbar mit dem Unterteil 6, der Platte 8 und dem Oberteil 13 verbunden ist, so daß bei geschlossenem Deckel Staub nicht in das Gerät eindringen kann.

## Patentansprüche

1. Kapselhalterung zum Einstecken und Fixieren von Arzneimittelkapseln, insbesondere in Inhalationsgeräten, bei der in einer Platte (2) eine Vertiefung (1) vorgesehen ist, dadurch gekennzeichnet, daß die Wandungen der Vertiefung parallel zur Längsachse einen größeren Abstand voneinander haben als den größten Kapseldurchmesser, wobei die Wandungen in ungleichem Abstand mindestens drei parallel zur Längsachse verlaufende Rippen (3; 3a; 3b; 3c) aufweisen, zwischen denen die Kapsel eingeklemmt wird, wobei die zur Längsachse orientierten Flächen oder Kanten der Rippen einen einbeschriebenen Zylinder (4) definieren, der einen geringfügig kleineren Durchmesser hat als das Kapselteil mit dem kleinsten Kapseldurchmesser.

2. Kapselhalterung nach Anspruch 1, in der die Vertiefung (1) zylindrisch ist und drei Rippen (3; 3a; 3b; 3c) aufweist, wobei zwei Rippen relativ nahe beieinander liegen und gegenüber der Mittellinie zwischen den beiden Rippen die dritte Rippe angeordnet ist.

3. Kapselhaltetung nach Anspruch 1 oder 2, bei welcher der Winkel α, der von den zu der Mitte der beiden nahe beieinander liegenden Rippen (3; 3a; 3b; 3c) verlaufenden Radien eingeschlossen wird, 30 bis 60, vorzugsweise 35 bis 50° beträgt.

4. Kapselhalterung nach Anspruch 3, bei welcher der Winkel α etwa 40° beträgt.

5. Kapselhalterung nach Anspruch 1 bis 4, dadurch gekennzeichnet, daß die Rippen (3; 3a; 3b; 3c) zur Mitte der Vertiefung hin abgerundet oder kantig sind.

6. Abwandlung der Kapselhalterung nach Anspruch 1 bis 5, dadurch gekennzeichnet, daß die Höhe der Rippen (3; 3a; 3b; 3c) nach unten zunimmt, so daß ihre zur Mittelachse gerichteten Flächen oder Kanten einen einbeschriebenen Kegelstumpf definieren, der an der Öffnungsseite der Vertiefung den größten Durchmesser hat.

7. Kapselhalterung nach einem der vorhergehenden Ansprüche, dadurch gekennzeichnet, daß die Rippen (3; 3b; 3c) an ihrem äußeren Ende abgerundet oder abgeschrägt sind.

## Claims

1. Capsule holder for the insertion and fixing of pharmaceutical capsules, particularly in inhalers, wherein there is provided, in a plate (2), a recess (1), characterised in that the walls of the recess parallel to the central axis are at a spacing from one another which is greater than the maximum capsule diameter, the walls having at least three ribs (3; 3a; 3b; 3c) running parallel to the central axis and at unequal spacings from one another, between which the capsule is gripped, the surfaces or edges of said ribs, oriented towards the longitudinal axis, define an inscribed cylinder (4) which is slightly smaller in diameter than the part of the capsule having the smallest diameter.

2. Capsule holder according to claim 1, in which the recess (1) is cylindrical and has three ribs (3; 3a; 3b; 3c), two ribs being located relatively close together and the third rib being arranged opposite the central line between the two ribs.

3. Capsule holder according to claim 1 or 2, wherein the angle α enclosed by the radii extending to the centre of the two adjacent ribs (3; 3a; 3b; 3c) is 30 to 60°, preferably 35 to 50°.

4. Capsule holder according to claim 3, wherein the angle α is about 40°.

5. Capsule holder according to claims 1 to 4, characterised in that the ribs (3; 3a; 3b; 3c) are rounded off or angular towards the centre of the recess.

6. Alternative form of the capsule holder according to claim 1 to 5, characterised in that the height of the ribs (3; 3a; 3b; 3c) increases downwardly, so that their surfaces or edges directed towards the central axis define an inscribed truncated cone which has its maximum diameter at the open end of the recess.

7. Capsule holder according to one of the preceding claims, characterised in that the ribs (3; 3b; 3c) are rounded off or chamfered at their outer ends.

## Revendications

1. Dispositif de retenue de capsules pour l'enfichage et la fixation de capsules de médicament, notamment dans des appareils d'inhalation, dans lequel un renfoncement (1) est prévu dans une plaque (2), caractérisé en ce que les parois du renfoncement sont séparées l'une de l'autre, parallèlement à l'axe longitudinal, par une distance supérieure au diamètre maximum des capsules, les parois possédant, à des distances différentes, au moins trois nervures (3 ; 3a ; 3b ; 3c) qui sont parallèles à l'axe longitudinal et entre lesquelles la capsule est serrée, les surfaces ou les bords des nervures, qui sont tournés vers l'axe longitudinal, définissent un cylindre inscrit (4), qui possède un diamètre nettement inférieur à celui de la partie de la capsule possédant le diamètre le plus petit.

2. Dispositif de retenue pour capsules selon la revendication 1, dans lequel le renfoncement (1) a une forme cylindrique et comporte trois nervures (3 ; 3a ; 3b ; 3c), deux nervures étant situées relativement près l'une de l'autre tandis que la troisième nervure est disposée à l'opposé de la nervure centrale entre les deux nervures.

3. Dispositif de retenue pour capsule selon la revendication 1 ou 2, dans lequel l'angle α, qui est formé par les rayons qui passent par le centre des deux nervures (3 ; 3a ; 3b; 3c) proches l'une de l'autre, est compris entre 30 et 60 et de préférence entre 35 et 50°.

4. Dispositif de retenue pour capsules selon la revendication 3, dans lequel l'angle α est égal à environ 40°.

5. Dispositif de retenue pour capsules selon les revendications 1 à 4, caractérisé en ce que les nervures (3 ; 3a ; 3b ; 3c) sont arrondies ou anguleuses en direction du centre du renfoncement.

6. Variante de réalisation du dispositif de retenue pour capsules selon les revendications 1 à 5, caractérisée en ce que la hauteur des nervures (3 ; 3a ; 3b ; 3c) augmente vers le bas de sorte que leurs surfaces ou bords, dirigés vers l'axe médian, définissent un tronc de cône inscrit, qui possède sont diamètre maximum au niveau du côté de l'ouverture du renfoncement.

7. Dispositif de retenue pour capsules selon l'une des revendications précédentes, caractérisé en ce que les nervures (3 ; 3a ; 3b ; 3c) sont arrondies ou biseautées au niveau de leur extrémité extérieure.
